# EUROPEAN PATENT APPLICATION

(11) **EP 4 253 369 A1**
(43) Date of publication of application: **04.10.2023**
(21) Application number: 22165671.3
(22) Date of filing: 30.03.2022
(51) Int. Cl.: C07D 261/04, A01N 43/80

(54) **CRYSTAL FORMS OF METHYL(1S,4R)-4-[[(5S)-3-(3,5-DIFLUOROPHENYL)-5-VINYL-4H-ISOXAZOLE-5-CARBONYL] AMINO]CYCLOPENT-2-ENE-1-CARBOXYLATE**

(71) Applicant: Bayer AG, 51373 Leverkusen (DE)
(72) Inventor: The designation of the inventor has not yet been filed
(74) Representative: BIP Patents

(57) **Abstract**

The present invention relates to the crystal forms of methyl(lS,4R)-4-[[(5S)-3-(3,5-difluorophenyl)-5-vinyl-4H-isoxazole-5-carbonyl]amino]cyclopent-2-ene-1-carboxylate **(I)**, to a method for preparing the crystal forms, to their use for preparing stable agrochemical formulations, and to their use in the field of agriculture for controlling harmful plants.

## Description

The present invention relates to the crystal forms of methyl(1S,4R)-4-[[(5S)-3-(3,5-difluorophenyl)-5-vinyl-4H-isoxazole-5-carbonyl]amino]cyclopent-2-ene-1-carboxylate **(I),** to a method for preparing the crystal forms, to their use for preparing stable agrochemical formulations, and to their use in the field of agriculture for controlling harmful plants. 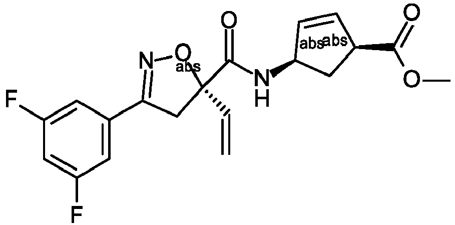 (**I**) "abs" means absolute configuration

### Scheme 1: Methyl(1S,4R)-4-[[(5S)-3-(3,5-difluorophenyl)-5-vinyl-4H-isoxazole-5-carbonyl]amino]cy-clopent-2-ene-1-carboxylate (I)

The herbicide methyl(1S,4R)-4-[[(5S)-3-(3,5-difluorophenyl)-5-vinyl-4H-isoxazole-5-carbonyl]amino]cy-clopent-2-ene-1-carboxylate **(I)** and a process for its production is published in WO2019/145245.

### Background

Polymorphism is the ability of a compound to crystallize in different crystalline phases with different arrangements and/or conformations of the molecules in the crystal lattice. Hence, polymorphs are different crystalline forms of the same pure chemical compound. On account of the different arrangement and/or conformation of molecules, polymorphs exhibit different physical, chemical and biological properties. Properties which may be affected include but are not limited solubility, dissolution rate, stability, optical and mechanical properties, etc. The relative stability of a polymorph depends on its free energy, i.e. a more stable polymorph has a lower free energy. Under a defined set of experimental conditions only one polymorph has the lowest free energy. This polymorph is the thermodynamically stable form and all other polymorph(s) is (are) termed metastable form(s). A metastable form is one that is thermodynamically unstable but can nevertheless be prepared, isolated and analyzed as a result of its relatively slow rate of transformation.

The occurrence of active substances in different polymorphic forms (hereinafter also named as polymorphs or crystalline forms) is of decisive importance for the production in industrial scale as well as for the development of formulations containing the active substance, as unwanted phase change can lead to thickening and potentially solidification of the formulation and/or large crystals, which can lead to blockages in application equipment, e.g. in spray nozzles in agricultural application machinery. The knowledge of the existence of crystalline modifications and their properties is thus of high relevance. Each polymorph is characterized by a specific, uniform packing and arrangement of the molecules in the solid state. Nevertheless, it is generally not predictable whether a given chemical compound forms polymorphs at all and if so, which physical and biological properties the different polymorphs may have.

Solid formulations (in which the active ingredient is present in solid form) are of great economic relevance since they may support the storage stability of the product and ensure consistent quality. These are, for example, granules, encapsulated granules, tablets, water-dispersible granules, water-dispersible tablets, water-dispersible powders or water-dispersible powders for the treatment of seed, dust formulations, formulations in which the active ingredient is present in dispersed form, such as, for example: suspension concentrates (SC), oil-based suspension concentrates, suspoemulsions or suspension concentrates for the treatment of seed.

However, this type of formulation can only be applied if the active ingredient is present in solid form, most preferably in the thermodynamically most stable modification.

Since the ecological and economic requirements placed on modern crop protection agents are continually increasing, for example as regards the storage stability, it would be desirable to have methyl(1S,4R)-4-[[(5S)-3-(3,5-difluorophenyl)-5-vinyl-4H-isoxazole-5-carbonyl]amino]cyclopent-2-ene-1-carboxylate (I) available in a crystalline form.

Methyl(1S,4R)-4-[[(5S)-3-(3,5-difluorophenyl)-5-vinyl-4H-isoxazole-5-carbonyl]amino]cyclopent-2-ene-1-carboxylate **(I)** crystallizes in a mixture of at least four different forms and -depending on the conditions-also solvates with very complex thermodynamic relations. This initiates precipitation of mixtures of the different forms in technical batches, which undergo thermodynamic transitions and circumvent consistend quality of the product.

Surprisingly, by applying very specific crystallization conditions, a new crystalline form of methyl(1S,4R)-4-[[(5S)-3-(3,5-difluorophenyl)-5-vinyl-4H-isoxazole-5-carbonyl]amino]cyclopent-2-ene-1-carboxylate **(I)** has now been found which avoids the aforementioned disadvantages of the thermodynamic transition at ambient conditions and supports storage stability as it can be provided in a solid formulation.

The present invention relates to the crystal forms of methyl(1S,4R)-4-[[(5S)-3-(3,5-difluorophenyl)-5-vinyl-4H-isoxazole-5-carbonyl]amino]cyclopent-2-ene-1-carboxylate (I), especially to the crystal form **D** of methyl(1S,4R)-4-[[(5S)-3-(3,5-difluorophenyl)-5-vinyl-4H-isoxazole-5-carbonyl]amino]cyclopent-2-ene-1-carboxylate (I).

The polymorphic form **D** of methyl(1S,4R)-4-[[(5S)-3-(3,5-difluorophenyl)-5-vinyl-4H-isoxazole-5-carbonyl]amino]cyclopent-2-ene-1-carboxylate can be characterized by X-ray powder diffractometry on the basis of the respective diffraction diagrams, which are recorded at 25°C and with Cu-Kα 1 radiation (1.5406 Å). The polymorphic form **D** according to the present invention displays at least 3, often at least 5, in particular at least 6, and especially all of the reflections quoted in the following as values:

**Table 1:**

| Reflections Modification/crystal form **D** (Peak maxima) [° 2θ] ±0.2° |
|---|
| 6.9 |
| 7.2 |
| 10.3 |
| 11.3 |
| 13.7 |
| 16.9 |
| 17.4 |
| 17.6 |
| 17.8 |
| 18.5 |
| 19.2 |
| 19.3 |
| 19.4 |
| 19.7 |
| 19.9 |
| 20.2 |
| 20.5 |
| 20.7 |
| 21.3 |
| 21.6 |
| 21.9 |
| 22.6 |
| 23.3 |
| 23.6 |
| 23.9 |
| 24.4 |
| 25.2 |
| 25.4 |
| 25.7 |
| 26.4 |
| 26.7 |
| 27.1 |
| 27.2 |
| 27.5 |
| 28.0 |
| 28.5 |
| 28.9 |
| 29.5 |
| 29.6 |
| 30.3 |
| 30.5 |
| 31.1 |
| 31.7 |
| 32.3 |
| 33.9 |
| 34.1 |
| 34.6 |
| 35.1 |
| 35.8 |
| 36.3 |

The crystal form/modification **D** of compound of formula **(I)** is characterized in that the X-ray powder diffractogram using Cu Kα radiation at 25°C has at least the following reflections: 17.4, 7.2, and 24.4, preferably at least the following reflections: 17.4, 7.2, 24.4 and 11.3, more preferably at least the following reflections: 17.4, 7.2, 24.4, 11.3 and 6.9, most preferably at least the following reflections: 17.4, 7.2, 24.4, 11.3, 6.9 and 16.9, each quoted as 2θ value ± 0.2°.

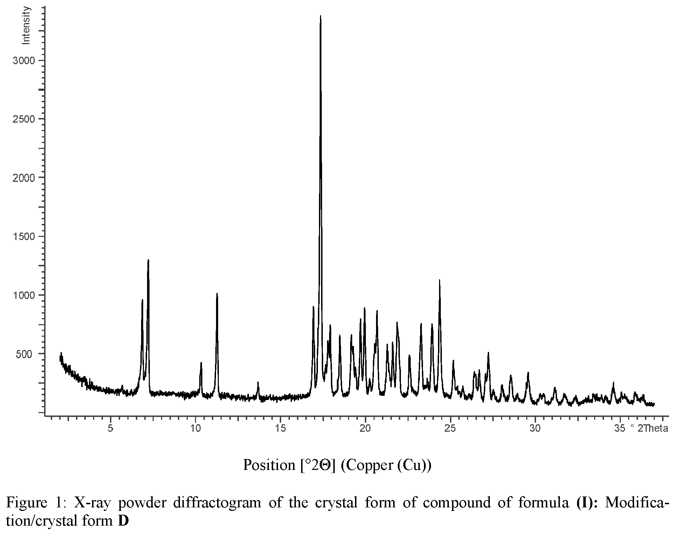

The polymorphic form **D** according to the present invention is further characterized by the X-ray powder diffractogram depicted in *Fig. 1**.*

Crystallization experiments reveal several further polymorphs of methyl(1S,4R)-4-[[(5S)-3-(3,5-difluorophenyl)-5-vinyl-4H-isoxazole-5-carbonyl]amino]cyclopent-2-ene-1-carboxylate **(I).**

The following crystal forms/modifications **A, B** and **C** can be identified:
The polymorphic forms **A, B** and **C** of methyl(1S,4R)-4-[[(5S)-3-(3,5-difluorophenyl)-5-vinyl-4H-isoxazole-5-carbonyl]amino]cyclopent-2-ene-1-carboxylate can be characterized by X-ray powder diffractometry on the basis of the respective diffraction diagrams, which are recorded at 25°C and with Cu-Kα 1 radiation (1.5406 Å). The polymorphic forms **A, B** and **C** display at least 3, often at least 5, in particular at least 6, and especially all of the reflections quoted in the following *Tables 2 to* 4 as values:

**Table 2:**

| Reflections modification/crystal form **A** (Peak maxima) [°2θ] ±0.2° |
|---|
| 7.1 |
| 7.9 |
| 9.9 |
| 11.9 |
| 12.5 |
| 13.2 |
| 15.8 |
| 16.9 |
| 17.4 |
| 17.9 |
| 18.2 |
| 19.1 |
| 19.8 |
| 20.2 |
| 20.6 |
| 21.1 |
| 21.4 |
| 21.7 |
| 22.8 |
| 23.1 |
| 23.5 |
| 23.9 |
| 24.1 |
| 24.5 |
| 25.5 |
| 25.7 |
| 26.5 |
| 26.7 |
| 27.1 |
| 28.4 |
| 29.0 |
| 29.3 |
| 30.1 |
| 31.4 |
| 31.7 |
| 32.4 |
| 33.3 |
| 33.9 |
| 34.6 |
| 35.2 |
| 35.4 |
| 35.8 |
| 36.0 |
| 36.8 |
| 37.2 |
| 37.9 |

The modification/crystal form **A** of compound of formula **(I)** is characterized in that the X-ray powder diffractogram using Cu Kα radiation at 25°C has at least the following reflections: 7.1; 18.2 and 23.5, preferably at least the following reflections: 7.1, 18.2, 23.5 and 11.9, more preferably at least the following reflections: 7.1, 18.2, 23.5, 11.9 and 17.9, most preferably at least the following reflections: 7.1, 18.2, 23.5, 11.9, 17.9 and 19.8, each quoted as 2θ value ± 0.2°.

**Table 3:**

| Reflections Modification/crystal form **B** (Peak maxima) [°2θ] ±0.2° |
|---|
| 6.9 |
| 7.2 |
| 10.3 |
| 11.3 |
| 13.1 |
| 13.6 |
| 16.1 |
| 16.8 |
| 17.3 |
| 17.8 |
| 17.9 |
| 18.4 |
| 18.7 |
| 19.5 |
| 20.0 |
| 20.7 |
| 21.5 |
| 21.9 |
| 22.6 |
| 23.2 |
| 23.9 |
| 24.4 |
| 25.1 |
| 26.4 |
| 26.7 |
| 27.0 |
| 27.3 |
| 27.5 |
| 28.1 |
| 28.6 |
| 29.3 |
| 29.5 |
| 30.1 |
| 31.1 |
| 31.7 |
| 32.3 |
| 33.4 |

The crystal form/modification **B** of compound of formula **(I)** is characterized in that the X-ray powder diffractogram using Cu Kα radiation at 25°C has at least the following reflections: 17.3; 6.9 and 19.5, preferably at least the following reflections: 17.3; 6.9, 19.5 and 17.9, more preferably at least the following reflections: 17.3; 6.9, 19.5, 17.9 and 17.8, most preferably at least the following reflections: 17.3; 6.9, 19.5, 17.9, 17.8 and 20.7, each quoted as 2θ value ± 0.2°.

**Table 4:**

| Reflections Modification/crystal form **C** (Peak maxima) [°2θ] ±0.2° |
|---|
| 6.1 |
| 6.8 |
| 7.2 |
| 7.9 |
| 10.3 |
| 11.3 |
| 12.3 |
| 13.0 |
| 13.7 |
| 15.7 |
| 15.9 |
| 16.1 |
| 17.1 |
| 17.4 |
| 17.8 |
| 18.5 |
| 18.9 |
| 19.2 |
| 19.5 |
| 19.8 |
| 20.2 |
| 20.7 |
| 21.0 |
| 21.4 |
| 21.7 |
| 22.3 |
| 22.6 |
| 22.8 |
| 23.3 |
| 23.6 |
| 23.9 |
| 24.1 |
| 24.4 |
| 24.7 |
| 25.1 |
| 25.6 |
| 25.9 |
| 26.8 |
| 27.2 |
| 27.7 |
| 28.5 |
| 29.5 |
| 30.2 |
| 31.8 |
| 33.4 |
| 34.3 |
| 34.5 |
| 35.9 |
| 36.3 |

The crystal form/modification C of compound of formula **(I)** is characterized in that the X-ray powder diffractogram using Cu Kα radiation at 25°C has at least the following reflections: 17.4; 19.8 and 7.9, preferably at least the following reflections: 17.4; 19.8, 7.9 and 17.1, more preferably at least the following reflections: 17.4; 19.8, 7.9, 17.1 and 20.2, most preferably at least the following reflections: 17.4; 19.8, 7.9, 17.1, 20.2 and 26.8, each quoted as 2θ value ± 0.2°.

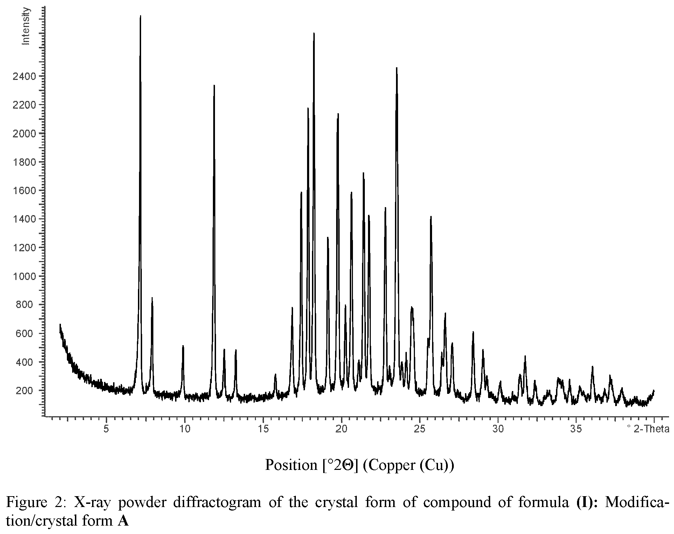

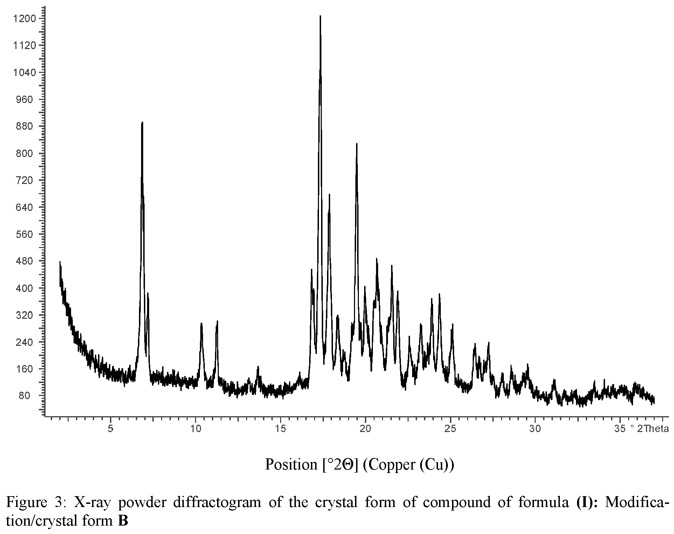

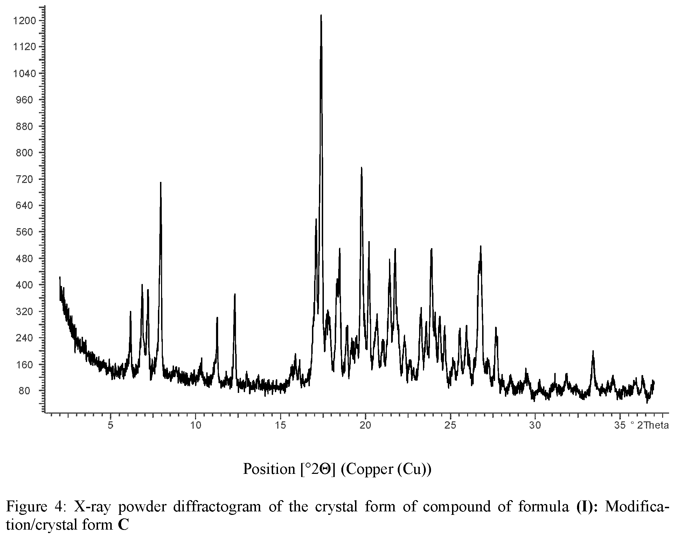

The polymorphic forms **A, B** and **C** are further characterized by the X-ray powder diffractogram depicted in *Fig. 2 to Fig. 4**.*

In a second embodiment, the present invention is directed to a process for the production of the polymorphic form **D,** comprising the following steps:
a) diluting and/or suspending the compound of formula (I) in a suitable solvent or solvent mixture;
b) heating the same to a temperature of at least 60°C; and
c) cooling the solution or slurry obtained in step b) to a temperature of less than 30°C.

The chemical preparation of methyl(1S,4R)-4-[[(5S)-3-(3,5-difluorophenyl)-5-vinyl-4H-isoxazole-5-carbonyl]amino]cyclopent-2-ene-1-carboxylate according to formula **(I)** is known from WO2019/145245. The compound of formula **(I)** as used in step a) can thus be prepared according to WO2019/145245, to which full reference is made hereby.

The compound of formula **(I)** in step a) can essentially be any known form of methyl(1S,4R)-4-[[(5S)-3-(3,5-difluorophenyl)-5-vinyl-4H-isoxazole-5-carbonyl]amino]cyclopent-2-ene-1-carboxylate. This means that methyl(1S,4R)-4-[[(5S)-3-(3,5-difluorophenyl)-5-vinyl-4H-isoxazole-5-carbonyl]amino]cyclopent-2-ene-1-carboxylate can be used in amorphous form or in a mixture of different polymorphic forms or in a mixture containing an amorphous and one or more different polymorphic forms.

Suitable solvents or solvent mixtures which can be used to dilute and/or suspend the compound of formula (I) in step a) and from which the compound of formula **(I)** is obtained in polymorphic form **D** in step c), are toluene, isopropanol or a 1 : 1 mixture of ethanol and water.

The solution of methyl(1S,4R)-4-[[(5S)-3-(3,5-difluorophenyl)-5-vinyl-4H-isoxazole-5-carbonyl]amino]cyclopent-2-ene-1-carboxylate according to formula **(I)** can also be prepared by transferring a reaction mixture obtained by chemical reaction, containing methyl(1S,4R)-4-[[(5S)-3-(3,5-difluorophenyl)-5-vinyl-4H-isoxazole-5-carbonyl]amino]cyclopent-2-ene-1-carboxylate according to formula **(I),** if necessary after removal of reagents and/or side products into a solvent or solvent mixture according to the present invention.

In step b) the solution or slurry is usually heated to a temperature of at least 60°C, preferably to a temperature of at least 70°C, and most preferably to a temperature of 80°C. In a preferred embodiment each solvent or solvent mixture is heated to its boiling temperature.

In step c) the solution or slurry is cooled to a temperature of less than 30°C, preferably less than 20°C, and preferably to a temperature of 10°C.

The crystallization of polymorphic form **D** can be promoted or accelerated by seeding with seed crystals of form **D.**

The isolation of the polymorphic form **D** from the mother liquid is effected by common techniques known in the art, for example by filtration, centrifugation or by decanting.

Alternatively, the polymorphic form **D** is isolated from the solvent or solvent mixture by allowing the solution or slurry to stand at the crystallization conditions of step c) until at least 90 wt.-% of the solvent or solvent mixture is evaporated.

The isolated polymorphic form **D** can optionally be washed with any solvent, preferably with the solvent or solvent mixture used for crystallization, with water or with a mixture of the solvent or solvent mixture and water. The washing step can optionally be repeated, whereby washing with water often is the last washing step. The washing is typically performed at temperatures below 30°C, often below 25°C and in particular below 20°C, optionally at 0 °C. In a further, optionally step, the crystals of polymorphic form **D** can be dried and then supplied for further processing.

By means of the crystallization according to the present invention, form **D** of methyl(1S,4R)-4-[[(5S)-3-(3,5-difluorophenyl)-5-vinyl-4H-isoxazole-5-carbonyl]amino]cyclopent-2-ene-1-carboxylate according to formula **(I)** is obtained with at least 85 %, in particular 90 %, and most preferably at least ≥ 95 %.

Thus, a particular embodiment of the present invention relates to methyl(1S,4R)-4-[[(5S)-3-(3,5-difluorophenyl)-5-vinyl-4H-isoxazole-5-carbonyl]amino]cyclopent-2-ene-1-carboxylate according to formula **(I),** which consists of at least 85% and often at least 90 % or at least ≥ 95 % of the polymorphic form **D.**

The content of form **D** according to the present invention is analyzed by Raman spectroscopy. Based on calculated electronically mixed Raman spectra (mixed by a software calculator in 5 % steps) a calibration curve, using a PLS regression, is generated. With this curve the proportional shares of the different forms are calculated. Due to the calibration accuracy the content of 100 % of polymorphic form **D** is not excluded by the above wording.

In a third embodiment, the present invention is directed to a plant protectionn agent in the form of customary formulations containing the polymorphic form **D** of methyl(1S,4R)-4-[[(5S)-3-(3,5-difluorophenyl)-5-vinyl-4H-isoxazole-5-carbonyl]amino]cyclopent-2-ene-1-carboxylate.

The plant protection agent may additionally comprise one or more further active substance(s) selected from the group consisting of herbicides, insecticides, acaricides, fungicides, safeners and/or plant growth regulator.

The plant protection agent may further comprise adjuvants which improve action, such as penetrants, e.g. vegetable oils, for example rapeseed oil, sunflower oil, mineral oils, for example paraffin oils, alkyl esters of vegetable fatty acids, for example rapeseed oil methyl ester or soya oil methyl ester, or alkanol alkoxylates and/or spreaders, for example alkylsiloxanes and/or salts, for example organic or inorganic ammonium or phosphonium salts, for example ammonium sulfate or diammonium hydrogenphosphate and/or retention promoters, for example dioctyl sulfosuccinate or hydroxypropylguar polymers and/or humectants, for example glycerol and/or fertilizers, for example ammonium-, potassium- or phosphorus-containing fertilizers.

Customary formulations are, for example, water-soluble liquids (SL), emulsion concentrates (EC), emulsions in water (EW), suspension concentrates (SC, SE, FS, OD), water-dispersible granules (WG), granules (GR) and capsule concentrates (CS); these and further possible formulation types are described, for example, by Crop Life International and in Pesticide Specifications, Manual on development and use of FAO and WHO specifications for pesticides, FAO Plant Production and Protection Papers - 173, prepared by the FAO/WHO Joint Meeting on Pesticide Specifications, 2004, ISBN: 9251048576.

Preference is given to formulations or use forms comprising auxiliaries, for example extenders, solvents, spontaneity promoters, carriers, emulsifiers, dispersants, frost protection agents, biocides, thickeners and/or further auxiliaries, for example adjuvants. An adjuvant in this context is a component which enhances the biological effect of the formulation, without the component itself having any biological effect. Examples of adjuvants are agents which promote retention, spreading, attachment to the leaf surface or penetration.

These formulations are prepared in a known way, for example by mixing the compounds of the formula (I) with auxiliaries such as, for example, extenders, solvents and/or solid carriers and/or other auxiliaries such as, for example, surfactants. The formulations are produced either in suitable facilities or else before or during application.

The auxiliaries used may be substances suitable for imparting special properties, such as certain physical, technical and/or biological properties, to the formulation of the compounds of the formula (I), or to the use forms prepared from these formulations (for example ready-to-use pesticides such as spray liquors or seed dressing products).

Suitable extenders are, for example, water, polar and nonpolar organic chemical liquids, for example from the classes of the aromatic and non-aromatic hydrocarbons (such as paraffins, alkylbenzenes, alkylnaphthalenes, chlorobenzenes), the alcohols and polyols (which, if appropriate, may also be substituted, etherified and/or esterified), the ketones (such as acetone, cyclohexanone), esters (including fats and oils) and (poly)ethers, the unsubstituted and substituted amines, amides, lactams (such as N-alkylpyrrolidones) and lactones, the sulfones and sulfoxides (such as dimethyl sulfoxide).

If the extender utilized is water, it is also possible to use, for example, organic solvents as auxiliary solvents. Useful liquid solvents are essentially: aromatics such as xylene, toluene or alkylnaphthalenes, chlorinated aromatics or chlorinated aliphatic hydrocarbons such as chlorobenzenes, chloroethylenes or methylene chloride, aliphatic hydrocarbons such as cyclohexane or paraffins, for example mineral oil fractions, mineral and vegetable oils, alcohols such as butanol or glycol and their ethers and esters, ketones such as acetone, methyl ethyl ketone, methyl isobutyl ketone or cyclohexanone, strongly polar solvents such as dimethylformamide and dimethyl sulfoxide, and water.

In principle, it is possible to use all suitable solvents. Examples of suitable solvents are aromatic hydrocarbons, such as xylene, toluene or alkylnaphthalenes, chlorinated aromatic or aliphatic hydrocarbons, such as chlorobenzene, chloroethylene or methylene chloride, aliphatic hydrocarbons, such as cyclohexane, paraffins, mineral oil fractions, mineral and vegetable oils, alcohols, such as methanol, ethanol, isopropanol, butanol or glycol and their ethers and esters, ketones such as acetone, methyl ethyl ketone, methyl isobutyl ketone or cyclohexanone, strongly polar solvents, such as dimethyl sulfoxide, and also water.

In principle, it is possible to use all suitable carriers. Useful carriers especially include: for example ammonium salts and ground natural minerals such as kaolins, clays, talc, chalk, quartz, attapulgite, montmorillonite or diatomaceous earth, and ground synthetic minerals such as finely divided silica, alumina and natural or synthetic silicates, resins, waxes and/or solid fertilizers. It is likewise possible to use mixtures of such carriers. Useful carriers for granules include: for example crushed and fractionated natural rocks such as calcite, marble, pumice, sepiolite, dolomite, and synthetic granules of inorganic and organic flours, and also granules of organic material such as sawdust, paper, coconut shells, corn cobs and tobacco stalks.

It is also possible to use liquefied gaseous extenders or solvents. Especially suitable are those extenders or carriers which are gaseous at standard temperature and under atmospheric pressure, for example aerosol propellants such as halogenated hydrocarbons, and also butane, propane, nitrogen and carbon dioxide.

Examples of emulsifiers and/or foam formers, dispersants or wetting agents having ionic or nonionic properties or mixtures of these surfactants are salts of polyacrylic acid, salts of lignosulfonic acid, salts of phenolsulfonic acid or naphthalenesulfonic acid, polycondensates of ethylene oxide with fatty alcohols or with fatty acids or with fatty amines, with substituted phenols (preferably alkylphenols or arylphenols), salts of sulfosuccinic esters, taurine derivatives (preferably alkyl taurates), phosphoric esters of polyethoxylated alcohols or phenols, fatty acid esters of polyols, and derivatives of the compounds containing sulfates, sulfonates and phosphates, for example alkylaryl polyglycol ethers, alkylsulfonates, alkyl sulfates, arylsulfonates, protein hydrolyzates, lignosulfite waste liquors and methylcellulose. The presence of a surfactant is advantageous if one of the compounds of the formula **(I)** and/or one of the inert carriers is insoluble in water and when the application takes place in water.

Further auxiliaries which may be present in the formulations and the use forms derived therefrom are dyes such as inorganic pigments, for example iron oxide, titanium oxide and Prussian Blue, and organic dyes such as alizarin dyes, azo dyes and metal phthalocyanine dyes, and nutrients and trace nutrients such as salts of iron, manganese, boron, copper, cobalt, molybdenum and zinc.

Additional components which may be present are stabilizers, such as cold stabilizers, preservatives, antioxidants, light stabilizers, or other agents which improve chemical and/or physical stability. Foam generators or antifoams may also be present.

In addition, the formulations and the use forms derived therefrom may also comprise, as additional auxiliaries, stickers such as carboxymethylcellulose and natural and synthetic polymers in the form of powders, granules or latices, such as gum arabic, polyvinyl alcohol and polyvinyl acetate, or else natural phospholipids such as cephalins and lecithins and synthetic phospholipids. Further auxiliaries may be mineral and vegetable oils.

It is possible if appropriate for still further auxiliaries to be present in the formulations and the use forms derived therefrom. Examples of such additives are fragrances, protective colloids, binders, adhesives, thickeners, thixotropic agents, penetrants, retention promoters, stabilizers, sequestrants, complexing agents, humectants, spreaders. In general, the compounds of the formula (I) can be combined with any solid or liquid additive commonly used for formulation purposes.

Useful retention promoters include all those substances which reduce dynamic surface tension, for example dioctyl sulfosuccinate, or increase viscoelasticity, for example hydroxypropylguar polymers.

Suitable penetrants in the present context are all those substances which are usually used for improving the penetration of agrochemical active compounds into plants. Penetrants are defined in this context by their ability to penetrate from the (generally aqueous) application liquor and/or from the spray coating into the cuticle of the plant and hence increase the mobility of the active compounds in the cuticle. The method described in the literature (Baur et al., 1997, Pesticide Science 51, 131-152) can be used for determining this property. Examples include alcohol alkoxylates such as coconut fatty ethoxylate (10) or isotridecyl ethoxylate (12), fatty acid esters, for example rapeseed oil methyl ester or soya oil methyl ester, fatty amine alkoxylates, for example tallowamine ethoxylate (15), or ammonium and/or phosphonium salts, for example ammonium sulfate or diammonium hydrogenphosphate.

In a preferred embodiment of the present invention, the plant protection agent contains more than 90 wt.%, and preferably more than 95 wt.%, of the polymorphic form **D** of the compound of the formula (I) based on the total amount of all forms of the compound of the formula (I) present in the composition.

It was now surprisingly found that the polymorphic form **D** has improved handling and formulation properties of formulations conating methyl(1S,4R)-4-[[(5S)-3-(3,5-difluorophenyl)-5-vinyl-4H-isoxazole-5-carbonyl]amino]cyclopent-2-ene-1-carboxylate. In a fourth embodiment, the present invention is therefore directed to the use of the polymorphic form **D** of the compound of formula (I) for the production of a formulation with increased storage stability.

In a fifth embodiment, the present invention is directed to the use of the polymorphic form **D** of the compound of formula (I) for combatting unwanted plant growth. In a particular embodiment, the present invention is directed to combatting unwanted plant growth in crops of useful plants. In a particular embodiment of the present invention, the useful plants are transgenic plants.

In a sixth embodiment, the present invention is directed to a method for combatting undesired plant growth, wherein the polymorphic form **D** or a plant protection agent as defined above containing the polymorphic form **D,** is applied to plants or the site of the unwanted vegetation.

### Working examples

### Methods

All data which is part of the present application has been prepared according to the methods described below unless otherwise indicated. The samples used for measurement were directly used and did not undergo any further sample preparation.

### XRPD

X-Ray diffraction patterns were recorded at room temperature using XRD -diffractometers X' Pert PRO (PAN-alytical) and STOE STADI-P (radiation Cu K alpha 1, wavelength 1.5406 Å). All X-Ray reflections are quoted as °2θ (theta) values (peak maxima) with a resolution of ± 0.2°.

### I Polymorphic form D of methyl(1S,4R)-4-[[(5S)-3-(3,5-difluorophenyl)-5-vinyl-4H-isoxazole-5-carbonyl]amino]cyclopent-2-ene-1-carboxylate according to formula (I)

### I.1 Preparation of polymorphic form D

### Examples 1.1 to 1.5

400 mg of methyl(1S,4R)-4-[[(5S)-3-(3,5-difluorophenyl)-5-vinyl-4H-isoxazole-5-carbonyl]amino]cyclo-pent-2-ene-1-carboxylate according to formula **(I)** were dissolved in an organic solvent according to Table 5 (below) under reflux. The solution was filtered and split into portions. These portions were allowed to crystallize under different conditions according to Table 5 (below).

The obtained crystals of polymorph **D** were isolated and analyzed by X-ray powder diffraction (XRPD).

**Table 5: Solvents used for crystallizing form D**

| Example | Solvent/Solvent Mixture | Crystallization temperature [°C] | *Special treatment* |
|---|---|---|---|
| 1.1 | 20 mL toluene | 25°C | Precipitation after addition of water/heptane |
| 1.2 | 20 mL isopropanol | 25°C | |
| 1.3 | 20 mL isopropanol | 25°C | Precipitation after addition of water/heptane |
| 1.4 | 30 mL 1 : 1 mixture of ethanol and water | 25°C | |
| 1.5 | 30 mL 1 : 1 mixture of ethanol and water | 25°C | Precipitation after addition of water/heptane |

### II Polymorphic form B of methyl(1S,4R)-4-[[(1S)-3-(3,5-difluorophenyl)-5-vinyl-4H-isoxazole-5-carbonyl]amino]cyclopent-2-ene-1-carboxylate according to formula (I)

### II.1 Preparation of polymorphic form B

400 mg of methyl(1S,4R)-4-[[(5S)-3-(3,5-difluorophenyl)-5-vinyl-4H-isoxazole-5-carbonyl]amino]cyclo-pent-2-ene-1-carboxylate according to formula (**I**) were dissolved in 30 mL of an 1 : 1 solvent mixture of ethanol and water under reflux. The solution was filtered and split into portions. One portion was stored in a refrigerator to fully drying resulting in the precipitation of crystal form B.

Another portion was stored in a fryer to fully drying leading to precipitation of crystal form B.

The obtained crystals of polymorph **B** were isolated and analyzed by X-ray powder diffraction (XRPD).

### III Polymorphic form C of methyl(1S,4R)-4-[[(5S)-3-(3,5-difluorophenyl)-5-vinyl-4H-isoxazole-5-carbonyl]amino]cyclopent-2-ene-1-carboxylate according to formula (I)

### III.1 Preparation of polymorphic form C

400 mg of methyl(1S,4R)-4-[[(5S)-3-(3,5-difluorophenyl)-5-vinyl-4H-isoxazole-5-carbonyl]amino]cyclo-pent-2-ene-1-carboxylate according to formula (**I**) were dissolved in 20 mL of isopropanol under reflux. The solution was filtered and split into portions. One portion was stored in a fryer to fully drying leading to precipitation of crystal form **C.**

The obtained crystals of polymorph **C** were isolated and analyzed by X-ray powder diffraction (XRPD).

## Claims

1. A crystalline form **D** of the compound of formula (I) 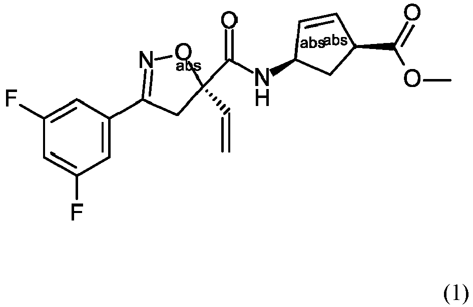 which in a X-ray powder diffractogram at 25°C and Cu-Kα 1 radiation displays at least 3 of the following reflections, quoted as 2θ value ± 0.2°:
| Reflections crystal form **D** (Peak maxima) [°2θ] ±0.2° |
|---|
| 6.9 |
| 7.2 |
| 10.3 |
| 11.3 |
| 13.7 |
| 16.9 |
| 17.4 |
| 17.6 |
| 17.8 |
| 18.5 |
| 19.2 |
| 19.3 |
| 19.4 |
| 19.7 |
| 19.9 |
| 20.2 |
| 20.5 |
| 20.7 |
| 21.3 |
| 21.6 |
| 21.9 |
| 22.6 |
| 23.3 |
| 23.6 |
| 23.9 |
| 24.4 |
| 25.2 |
| 25.4 |
| 25.7 |
| 26.4 |
| 26.7 |
| 27.1 |
| 27.2 |
| 27.5 |
| 28.0 |
| 28.5 |
| 28.9 |
| 29.5 |
| 29.6 |
| 30.3 |
| 30.5 |
| 31.1 |
| 31.7 |
| 32.3 |
| 33.9 |
| 34.1 |
| 34.6 |
| 35.1 |
| 35.8 |
| 36.3 |

2. The form **D** of the compound of claim 1, which in a X-ray powder diffractogram at 25°C and with Cu-Kα 1 radiation displays at least the following reflections, quoted as 2θ value ± 0.2°: 17.4, 7.2 and 24.4.

3. The form **D** of the compound of claim 1, which in a X-ray powder diffractogram at 25°C and with Cu-Kα 1 radiation displays at least the following reflections, quoted as 2θ value ± 0.2°: 17.4, 7.2, 24.4 and 11.3.

4. The form **D** of the compound of claim 1, which in a X-ray powder diffractogram at 25°C and with Cu-Kα 1 radiation displays at least the following reflections, quoted as 2θ value ± 0.2°: 17.4, 7.2, 24.4, 11.3 and 6.9.

5. The form **D** of the compound of claim 1, which in a X-ray powder diffractogram at 25°C and with Cu-Kα 1 radiation displays at least the following reflections, quoted as 2θ value ± 0.2°: 17.4, 7.2, 24.4, 11.3, 6.9 and 16.9.

6. A process for the production of crystalline form **D** according to any of claims 1 to 5, comprising the following steps:
a) diluting and/or suspending the compound of formula (I) in a suitable solvent or solvent mixture;
b) heating the same to a temperature of at least 60°C; and
c) cooling the solution or slurry obtained in step b) to a temperature of less than 30°C.

7. The process according to claim 6, wherein the solvent or solvent mixture of step a) is selected from the group consisting of toluene, isopropanol or a 1 : 1 mixture of ethanol and water.

8. The process according to claim 6 or 7, wherein the heating temperature of step b) is at least 70°C.

9. The process according to any of claims 6 to 8, wherein the solution or slurry of step c) is cooled to a temperature of less than 20°C.

10. A plant protection agent containing the crystalline form **D** of formula (1) according to any of claims 1 to 5.

11. The plant protection agent according to claim 10, which further comprises one or more agriculturally acceptable additive customary for the formulation of plant protection agents.

12. The plant protection agent according to claim 10 or 11, which further comprises one or more additional active substance(s) selected from the group consisting of herbicides, insecticides, acaricides, fungicides, safeners and/or plant growth regulator.

13. Use of the polymorphic form **D** according to any of claims 1 to 5 or of a plant protection agent according to any of claims 10 to 12 for the production of a formulation with improved storage stability.

14. Use of the polymorphic form **D** according to any of claims 1 to 5 or of a plant protection agent according to any of claims 10 to 12 for combatting undesired plant growth

15. Use according to claim 13 or 14, wherein the polymorphic form **D** is used for combatting unwanted plant growth in crops of useful plants.

16. Method for combating undesired plant growth, wherein the polymorphic form **D** according to any of claims 1 to 5 or a plant protection agent according to any of claims 10 to 12 is applied to plants or the site of the unwanted vegetation.
